Europäisches Patentamt

European Patent Office

Office européen des brevets

(11) Numéro de publication : **0 296 917 B1**

(12) **FASCICULE DE BREVET EUROPEEN**

(45) Date de publication du fascicule du brevet :
**04.03.92 Bulletin 92/10**

(51) Int. Cl.$^5$ : **G01N 1/10,** G21F 7/06

(21) Numéro de dépôt : **88401397.0**

(22) Date de dépôt : **08.06.88**

(54) **Dispositif de mise en circulation continue d'un liquide en vue d'effectuer un prélèvement ou un contrôle de ce liquide.**

(30) Priorité : **12.06.87 FR 8708217**

(43) Date de publication de la demande :
**28.12.88 Bulletin 88/52**

(45) Mention de la délivrance du brevet :
**04.03.92 Bulletin 92/10**

(84) Etats contractants désignés :
**DE GB**

(56) Documents cités :
**EP-A- 0 078 211**
**EP-A- 0 079 283**
**EP-A- 0 155 107**
**US-A- 3 255 575**

(73) Titulaire : **COMMISSARIAT A L'ENERGIE
ATOMIQUE Etablissement de Caractère
Scientifique Technique et Industriel
31/33, rue de la Fédération
F-75015 Paris (FR)**

(72) Inventeur : **Farrugia, Jean-Marie
19, Boulevard de l'Orient
F-30133 Les Angles (FR)**
Inventeur : **Delagrange, Jacques
Villa 8-Paniscoule
F-30200 Bagnols sur Ceze (FR)**

(74) Mandataire : **Mongrédien, André et al
c/o BREVATOME 25, rue de Ponthieu
F-75008 Paris (FR)**

EP 0 296 917 B1

**Description**

La présente invention a pour objet un dispositif de mise en circulation continue d'un liquide et plus particulièrement de mise en circulation de ce liquide entre une cuve de stockage et un récipient où doit être effectué un prélèvement ou un contrôle.

Dans l'industrie nucléaire notamment, il existe à l'heure actuelle un certain nombre de techniques permettant de prélever des échantillons de liquide radioactif. Par exemple, on peut utiliser une aiguille creuse plongeant directement au sein de la masse de liquide dont on veut extraire un échantillon. Dans d'autres systèmes, l'aiguille creuse plonge au sein d'une capacité intermédiaire remplie au préalable du liquide à tester par un système de transfert qui peut être un système à air-lift ou par mise sous vide. Enfin, le prélèvement peut être effectué dans une capacité ou un récipient balayé par la solution à échantillonner, le liquide étant mis en circulation au moyen d'un système de type air-lift. Cette dernière méthode permet en général de ne pas avoir de rétention de liquide dans le circuit de prélèvement. Le prélèvement est effectué au moyen d'un cruchon préalablement mis sous vide et fermé par un obturateur souple, ce dernier étant piqué sur l'aiguille creuse et percé par celle-ci.

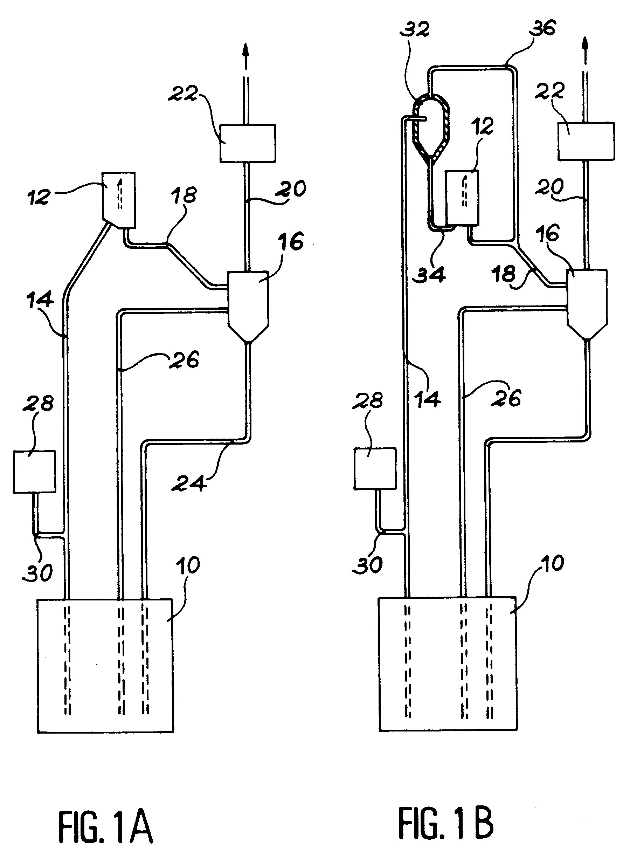

La figure 1A ci-jointe illustre un dispositif de mise en circulation continue de liquide pour la mise en oeuvre d'une méthode de ce type. On voit que le liquide à tester est contenu dans une cuve 10 reliée à un dispositif de prélèvement 12 par une conduite d'alimentation 14. Le dispositif de prélèvement 12 se trouve au-dessus de la cuve 10, et en tout cas à un niveau plus élevé que celui de la surface libre du liquide contenu dans la cuve. Le dispositif comporte en outre un séparateur 16 relié au dispositif de prélèvement 12 par une canalisation 18. Cette dernière débouche dans le séparateur 16 à la partie supérieure de celui-ci, qui est située à un niveau plus bas que le dispositif de prélèvement 12. Une conduite 20 équipée d'un dévésiculeur 22 met la partie supérieure du séparateur 16 en communication avec un appareil permettant de créer une dépression, par exemple une pompe à vide (cet appareil n'est pas représenté sur la figure 1A). Une conduite de retour 24 relie la partie inférieure du séparateur 16 à la cuve 10, ce qui permet le retour du liquide dans la cuve. Eventuellement, on peut prévoir une conduite de trop-plein 26 reliant le séparateur 16 à la cuve 10. Le dispositif comporte en outre une source d'air comprimé 28 reliée par une conduite 30 à la conduite d'alimentation 14.

Le fonctionnement d'un tel dispositif de mise en circulation est le suivant :

Lorsque l'appareil de mise en dépression ou la pompe à vide est mis en marche, ceci crée une dépression dans le dispositif et notamment dans la conduite d'alimentation 14. Le liquide contenu dans la cuve 10 monte donc dans cette conduite. La source d'air comprimé 28 est mise en route de manière à insuffler l'air comprimé, à travers la conduite 30, dans la conduite 14 et dans la masse de liquide contenue dans celle-ci. Comme l'appareil de mise en dépression est toujours en marche, il se crée dans la conduite 14 des bulles qui montent et entraînent le liquide. Celui-ci passe dans le dispositif de prélèvement 12 où se trouve une aiguille creuse dont la partie inférieure est à l'intérieur du liquide. Ce dernier descend ensuite du système de prélèvement 12 au séparateur 16 par gravité le long de la canalisation 18. C'est en fait un mélange de gaz et de liquide qui arrive dans le séparateur. Comme celui-ci est relié à l'appareil de mise en dépression par la conduite 20, le gaz est extrait par cette conduite tandis que le liquide se rassemble à la partie inférieure du séparateur 16 et retombe par gravité dans la cuve 10 en suivant la conduite 24. Quant au dévésiculeur 22, il a pour rôle de permettre la séparation des gouttelettes qui auraient pu être entraînées avec le gaz le long de la conduite 20.

Un tel dispositif présente des inconvénients dus au fait que ce n'est pas du liquide pur, mais en fait un mélange de liquide et de gaz qui circule à travers le liquide de prélèvement 12. Ceci perturbe le prélèvement dans le cruchon en créant des aspirations d'air parasites. De plus, l'existence d'une pression réduite diminue les performances des prélèvements effectués à l'ai d'un cruchon préalablement mis sous vide par suite de la diminution de la différence de pression entre le cruchon et le circuit de liquide. Chaque cruchon étant rempli partiellement, pour un volume constant de solution prélevée, la durée d'utilisation du banc de prélèvement et le volume de déchets induits sont plus importants que si les cruchons étaient remplis au maximum.

La présente invention a pour but de remédier à ces inconvénients en proposant un dispositif de mise en circulation continue d'un liquide permettant de prélever, au niveau de l'aiguille creuse, un liquide débarrassé au maximum du gaz d'entrainement du système à air-lift.

D'autre part, la demande de brevet européen EP-A-0 079 283 décrit également un système de mise en circulation continue d'un liquide, en vue d'un prélèvement ou d'un contrôle de ce liquide. Il présente des éléments communs avec le dispositif selon l'invention. Ces mêmes éléments sont les suivants :
  – une cuve dans laquelle se trouve le liquide ;
  – une source de pression ;
  – une conduite dans laquelle débouche la source de pression ;
  – un séparateur de l'air contenu dans le liquide ;
  – un dévésiculeur ;
  – une source de dépression aboutissant dans la partie supérieure du séparateur.

Ce dispositif, servant à la mise en circulation continue d'un liquide entre une cuve de stockage et un dispositif de prélèvement situé à un niveau supérieur à celui de la surface libre du liquide contenu dans la cuve, comprend, de manière connue :

– une conduite d'alimentation pour le transport du liquide entre la cuve de stockage et le dispositif de prélèvement ;

– une source de gaz comprimé ;

– une conduite reliant cette source de gaz comprimé à la conduite d'alimentation ;

– un séparateur ayant une partie supérieure et une partie inférieure, le dispositif de prélèvement se trouvant à un niveau plus élevé que le séparateur ;

– une canalisation reliant le dispositif de prélèvement au séparateur,

– un appareil permettant de créer une dépression,

– une conduite, équipée d'un dévésiculeur, reliant la partie supérieure du séparateur à cet appareil permettant de créer une dépression, et

– une conduite de retour reliant la partie inférieure du séparateur à ladite cuve.

Selon l'invention, le dispositif comporte en outre :

– une capacité intermédiaire constituée par un récipient fermé, cette capacité intermédiaire ayant une partie supérieure et une partie inférieure séparées par une partie intermédiaire, la conduit d'alimentation débouchant à l'intérieur de cette capacité intermédiaire dans sa partie intermédiaire, la partie inférieure de cette capacité intermédiaire se trouvant à un niveau plus élevé que le dispositif de prélèvement,

– une conduite reliant la partie inférieure de cette capacité intermédiaire au dispositif de prélèvement, et

– une conduite mettant la partie supérieure de la capacité intermédiaire en communication avec l'appareil permettant de créer une dépression.

Dans un mode de réalisation préféré, cette conduite débouche dans la canalisation qui relie le dispositif de prélèvement au séparateur.

Cette disposition permet de faire circuler, dans le dispositif de prélèvement, un liquide débarrassé au moins en grande partie de l'air moteur. En effet, comme la conduite d'alimentation débouche dans la capacité intermédiaire, le liquide tombe au fond de celle-ci, tandis que le gaz s'en sépare et se rassemble à la partie supérieure. Il en est extrait par la conduite qui met la partie supérieure de cette capacité intermédiaire en communication avec l'appareil de mise en dépression. C'est donc du liquide débarrassé de la plus grande partie du gaz porteur qui arrive au dispositif de prélèvement, ce qui permet un meilleur remplissage des cruchons.

Selon un autre aspect du dispositif de mise en circulation, objet de l'invention, le dispositif de prélèvement comprend au moins une aiguille creuse montée sur un embout lui-même disposé sur un support comprenant une conduite d'arrivée et une conduite de sortie du liquide à échantillonner entre lesquelles se trouve une capacité intermédiaire, la partie inférieure de ladite capacité intermédiaire étant reliée à la conduite d'arrivée par un conduit de vidange, la partie inférieure de l'embout ayant la forme d'un manchon plongeant dans ladite capacité intermédiaire et ouvert à son extrémité inférieure, ledit support présentant en outre un orifice mettant en communication la conduite d'arrivée, la conduite de sortie et le conduit de vidange à un niveau inférieur à celui de la partie inférieure de la capacité intermédiaire.

Dans un autre mode de réalisation de ce dispositif de mise en circulation de liquide, le dispositif de prélèvement comprend :

– au moins une aiguille creuse montée sur un embout lui-même disposé sur un support ;

– une conduite sensiblement verticale ayant une extrémité supérieure reliée audit support et une extrémité inférieure ; et

– une capacité apte à retenir une certaine quantité du liquide à échantillonner, la partie inférieure de ladite conduite débouchant dans cette capacité, celle-ci étant en communication avec les conduites reliant le dispositif de prélèvement à la capacité intermédiaire d'une part et au séparateur d'autre part, une partie de l'aiguille creuse se trouvant dans ladite conduite et l'extrémité inférieure de cette aiguille se trouvant dans la masse de liquide contenue dans ladite capacité lorsque l'embout est monté sur le support.

L'invention apparaîtra mieux à la lecture de la description qui va suivre, donnée à titre d'exemple purement illustratif en référence aux dessins annexés dans lesquels :

– la figure 1A est une vue schématique en élévation d'un dispositif de mise en circulation de liquide selon l'art antérieur ;

– la figure 1B est une vue schématique semblable à la figure 1A illustrant un dispositif de mise en circulation d'un liquide conforme à l'invention ;

– la figure 2 est une vue schématique en coupe verticale d'un dispositif de prélèvement selon l'art antérieur ;

– la figure 3 est une vue schématique semblable à la figure 2 illustrant un dispositif de prélèvement utilisable avec le dispositif de mise en circulation selon la présente invention ;

– la figure 4 est une vue schématique en coupe verticale d'un autre mode de réalisation d'un dispositif de prélèvement utilisable avec le dispositif de mise en circulation conforme à l'invention ;

– la figure 5A est une vue semblable à la figure 1B montrant comment le dispositif de prélèvement selon la figure 4 peut être monté dans le dispositif de mise en circulation en amont du séparateur ; et

– la figure 5B est une vue semblable à la figure 5A montrant comment le dispositif de prélèvement peut être monté dans le dispositif de mise en circulation en aval du séparateur.

La figure 1B représente un dispositif de mise en circulation d'un liquide selon l'invention dans lequel on retrouve la plupart des éléments du dispositif illustré à la figure 1A. Pour plus de clarté, les éléments semblables portent les mêmes numéros de référence.

Selon l'invention, le dispositif de la figure 1B comporte une capacité intermédiaire 32 dont la partie inférieure est située à un niveau plus élevé que le dispositif de prélèvement 12. La conduite d'alimentation 14 débouche, non pas directement dans le dispositif 12, comme dans le cas de la figure 1A, mais à l'intérieur de la capacité intermédiaire 32, sensiblement au milieu de celle-ci. Une première conduite 34 relie la partie inférieure de la capacité 32 au dispositif de prélèvement 12 tandis qu'une deuxième conduite 36 relie la partie supérieure de la capacité 32 à la canalisation 18 qui permet le passage du liquide du dispositif de prélèvement 12 au séparateur 16. Tous les autres éléments sont identiques à ceux du dispositif illustré à la figure 1A.

Le fonctionnement du dispositif illustré à la figure 1B est le suivant :

Lorsque le dispositif de mise en dépression ou la pompe évide est mis en marche, une dépression est créée à l'intérieur du circuit et le liquide contenu dans la cuve 10 s'élève dans la conduite d'alimentation 14. Comme précédemment, la source d'air comprimé 28 est mise en route et de l'air comprimé est insufflé, à travers la conduite 30, dans le liquide contenu dans la conduite 14. Ceci a pour effet d'entraîner le liquide de bas en haut le long de cette conduite, mais le mélange air-liquide arrive dans la capacité intermédiaire 32 au lieu d'arriver directement dans le dispositif de prélèvement 12. Comme la partie supérieure de la capacité 32 est reliée au dispositif de mise en dépession par les conduites 36, 18, le séparateur 16 et la conduite 20, une grande partie du gaz est extraite par la partie supérieure de la capacité intermédiaire 32. C'est donc un liquide débarrassé de la plus grande partie du gaz d'entraînement qui tombe dans la conduite 34 et arrive au dispositif de prélèvement 12. Le gaz aspiré par la conduite 36 se remélange au liquide dans la conduite 18, mais le gaz est à nouveau extrait lorsque le mélange arrive dans le séparateur 16.

Une telle disposition améliore notablement l'efficacité du prélèvement car c'est du liquide dégazé qui arrive au système de prélèvement 12. D'autre part, comme la plus grande partie de l'air moteur est extraite à la partie supérieure de la capacité intermédiaire 32, il n'y a pratiquement plus d'air moteur mélangé au liquide et la différence de pression entre le dispositif de prélèvement et les cruchons est maintenue à un niveau élevé, ce qui permet un meilleur remplissage des cruchons.

L'invention a encore pour objet une amélioration de l'efficacité de tels dispositifs grâce à une construction particulière du dispositif de prélèvement 12.

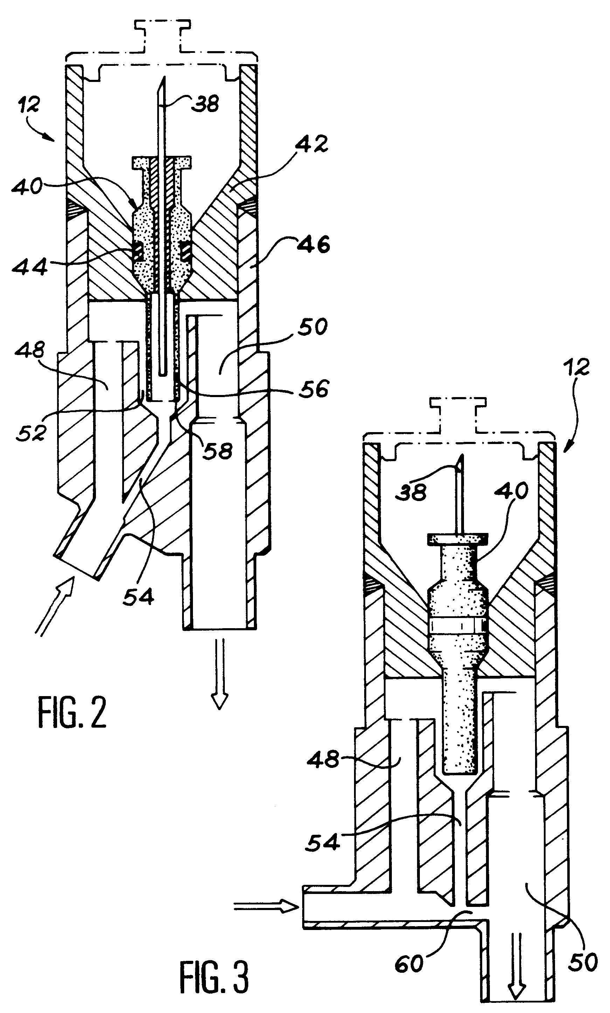

La figure 2 illustre un dispositif de prélèvement selon la demande de brevet européen EP-A-0 078 211. Ce dispositif comprend une aiguille creuse 38 montée sur un embout 40 lui-même disposé à l'intérieur d'un bouchon 42 : un joint torique 44 assure l'étanchéité entre l'embout 40 et le bouchon 42. Ce dernier est lui-même monté sur un support 46 muni d'une conduite d'arrivée 48 et d'une conduite de départ 50 du liquide à échantillonner. Une capacité intermédiaire 52 est disposée au centre du support 46 entre la conduite d'arrivée 48 et la conduite de sortie 50. Sur la figure, on voit encore un conduit de vidange 54 de faible diamètre qui est fortement incliné et qui relie le fond de la capacité intermédiaire 52 à la conduite d'arrivée 48. L'embout 40 est prolongé à sa partie inférieure par un manchon cylindrique 56 dont la longueur est à peu près égale à celle de la capacité intermédiaire 52 et ce manchon est ouvert à son extrémité inférieure 58.

Lorsqu'on met en circulation le liquide à analyser à l'aide du dispositif illustré à la figure 1B, ce liquide arrive dans la capacité intermédiaire 52 aussi bien par la partie supérieure de celle-ci à travers la conduite d'entrée 48 que par la partie inférieure grâce au conduit 54. Cette disposition permet un renouvellement et une homogénéisation constants du liquide pendant toute la durée du prélèvement. Le manchon 56 a un rôle de tranquilliseur afin d'éviter les turbulences éventuelles provoquées à l'intérieur de la capacité 52 par la rentrée d'air due à la dépression du système d'air-lift. Ces turbulences risqueraient de provoquer des vibrations inadmissibles sur l'aiguille 38 de prélèvement. Quant au conduit 15, outre le fait qu'il assure une bonne homogénéisation du liquide en lui permettant d'arriver dans la capacité intermédiaire par deux endroits différents, il assure une vidange efficace lors de l'arrêt de l'installation grâce à sa très forte inclinaison.

Cependant, on peut encore améliorer l'efficacité de vidange d'un tel dispositif grâce à la construction illustrée à la figure 3. Le dispositif de prélèvement illustré sur cette figure est semblable à celui qui est illustré à la figure 2, mais le conduit de vidange 54 est vertical au lieu d'être incliné tandis qu'un orifice 60 permet de mettre en communication la conduite d'arrivée 48, le conduit de vidange 54 et la conduite de départ du liquide 50. Cette disposition permet de déboucher les lignes de vidange du dispositif de prélèvement en introduisant par celui-ci un dispositif d'injection de fluide sous pression. De plus, cette disposition rend le système auto-nettoyant par simple mise sous vide sans injection d'air moteur dans le système d'air-lift. En effet, ce nettoyage est réalisé par le courant gazeux créé par la mise sous vide, courant gazeux qui balaye énergiquement toute la tuyauterie.

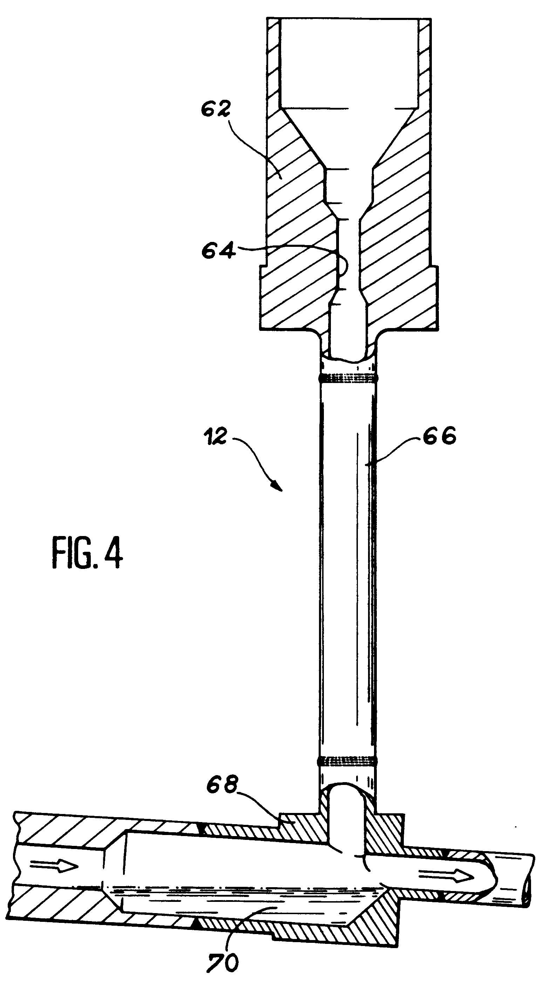

La figure 4 illustre un autre mode de réalisation d'un dispositif de prélèvement utilisable dans l'invention.

Ce dispositif comporte essentiellement un support 62 percé d'un passage 64. L'embout portant l'aiguille creuse (non représenté sur la figure 4) est placé sur ce support dans le passage 64. le support 62 est prolongé à sa partie inférieure par une conduite sensiblement verticale de grande longueur 66 (la longueur de cette conduite peut être par exemple de l'ordre de 500 à 700 millimètres). La partie inférieure de la conduite 66 débouche dans une capacité 68 ayant une forme telle qu'elle puisse retenir une certaine quantité du liquide à échantillonner 70. Cette capacité 68 se trouve à un niveau plus bas que le support 62 et la conduite 66 est placée sensiblement verticalement. Dans cette variante, l'aiguille creuse montée sur l'embout a une grande longueur, c'est-à-dire que sa partie inférieure se trouve dans la masse liquide 70 lorsque l'embout est monté sur le support 62. Une telle disposition permet d'effectuer un prélèvement même lorsque le système d'air-lift est à l'arrêt. En effet, du fait de la présence d'une réserve 70 de liquide dans la capacité 68, ce dernier est aspiré le long de l'aiguille creuse dès que l'on pique un cruchon sur celle-ci.

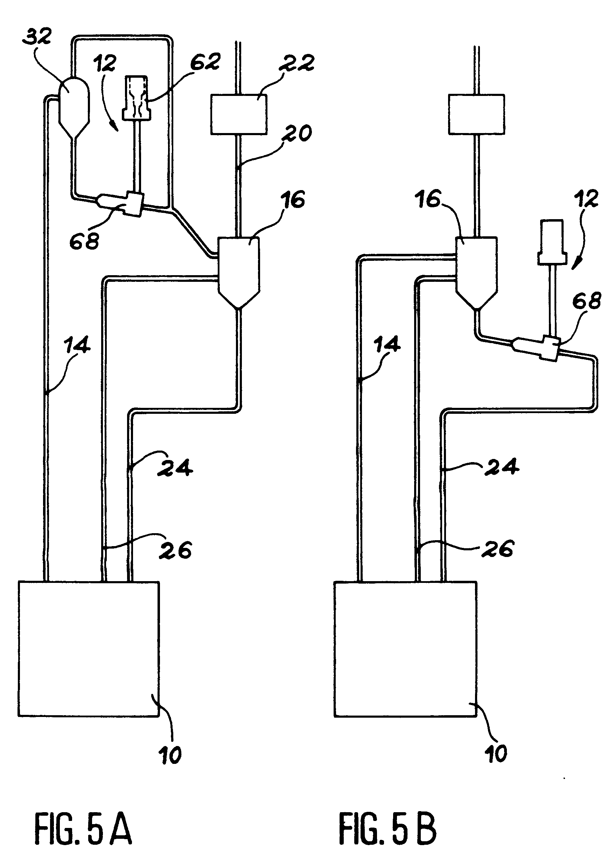

Un dispositif de prélèvement tel que celui qui est illustré à la figure 4 peut être placé dans le dispositif de prélèvement objet de l'invention aussi bien en amont qu'en aval du pot séparateur comme cela est illustré aux figures 5A et 5B.

Dans le cas de la figure 5A, il est placé exactement comme le dispositif 12 de la figure 1B entre la capacité intermédiaire 32 et le séparateur 16. Dans le cas de la figure 5B, il est placé en aval du séparateur 16. Dans ce cas, la capacité intermédiaire 32 est supprimée puisque la séparation entre le liquide et le gaz moteur est effectuée dans le séparateur, le liquide dégazé s'écoulant par gravité jusqu'à la capacité 68. Dans le cas de la figure 5B, il faut veiller à ce que l'extrémité supérieure de l'aiguille creuse se trouve au-dessus du niveau auquel le tuyau de trop-plein 26 débouche dans le séparateur 16.

L'utilisation d'un dispositif de prélèvement tel que celui qui est illustré aux figures 4, 5A et 5B est surtout intéressante dans le cas où il est nécessaire d'utiliser un système d'air-lift sous un vide élevé, car un tel dispositif permet de s'affranchir de la perte d'efficacité du remplissage des cruchons due à une diminution de la différence de pression entre le cruchon et le circuit du liquide à échantillonner. En effet, comme une certaine masse de liquide est retenue dans la capacité 68, le liquide peut être aspiré dans le cruchon même après arrêt du système d'air-lift. Le volume de cette capacité peut être déterminé afin que la quantité de liquide retenue permette de remplir plusieurs cruchons. Lorsque le dispositif de prélèvement illustré à la figure 4 est placé en aval du séparateur, comme cela est illustré à la figure 5B, on peut utiliser un système d'air-lift à submergence naturelle au lieu d'un système d'air-lift sous vide.

Ainsi, le dispositif objet de l'invention présente des avantages particulièrement intéressants puisqu'il permet une meilleure efficacité de prélèvement du liquide à échantillonner grâce à un dégazage de celui-ci effectué en amont du dispositif de prélèvement. De plus, il permet une vidange et un nettoyage plus efficaces que dans les dispositifs de l'art antérieur.

Enfin, il est bien entendu que l'invention ne se limite pas au seul mode de réalisation qui vient d'être décrit ici, mais qu'on peut envisager des variantes de construction sans sortir pour autant du cadre de l'invention. Par exemple si, dans le mode de réalisation préféré, la conduite 36 débouche dans la canalisation 18, on ne sortirait pas du cadre de l'invention en la faisant déboucher en un autre endroit du circuit, pourvu qu'elle soit en communication avec l'appareil de mise en dépression.

## Revendications

1. Dispositif de mise en circulation continue d'un liquide entre une cuve de stockage (10) et un dispositif de prélèvement (12) situé à un niveau supérieur à celui de la surface libre du liquide contenu dans la cuve, comprenant :
   - une conduite d'alimentation (14) pour le transport du liquide entre la cuve de stockage (10) et le dispositif de prélèvement (12),
   - une source de gaz comprimé (28),
   - une conduite (30) reliant cette source de gaz comprimé (28) à la conduite d'alimentation (14),
   - un séparateur (16) ayant une partie supérieure et une partie inférieure, le dispositif de prélèvement (12) se trouvant à un niveau plus élevé que le séparateur (16),
   - une canalisation (18) reliant le dispositif de prélèvement (12) au séparateur (16),
   - un appareil permettant de créer une dépression,
   - une conduite (20), équipée d'un dévésiculeur (22), reliant la partie supérieure du séparateur (16) à cet appareil permettant de créer une dépression, et
   - une conduite de retour (24) reliant la partie inférieure du séparateur (16) à ladite cuve (10), caractérisé en ce qu'il comporte en outre :
   - une capacité intermédiaire (32 ) constituée par un récipient fermé, cette capacité intermédiaire (32) ayant une partie supérieure et une partie inférieure séparées par une partie intermédiaire, la conduite d'alimentation (14) débouchant à l'intérieur de cette capacité intermédiaire (32) dans sa partie intermédiaire, la partie inférieure de cette capacité intermédiaire (32) se trouvant à un niveau plus élevé que le dispositif de prélèvement (12),

– une conduite (34) reliant la partie inférieure de cette capacité intermédiaire (32) au dispositif de prélèvement (12), et

– une conduite (36) mettant la partie supérieure de la capacité intermédiaire (32) en communication avec l'appareil permettant de créer une dépression.

2. Dispositif selon la revendication 1, caractérisé en ce que ladite conduite (36) mettant la partie supérieure de la capacité intermédiaire (32) en communication avec l'appareil permettant de créer une dépression débouche dans ladite canalisation (18) qui relie le dispositif de prélèvement (12) au séparateur (16).

3. Dispositif selon la revendication 1, caractérisé en ce que le dispositif de prélèvement comprend au moins une aiguille creuse (38) montée sur un embout (40) lui-même disposé sur un support (46) comprenant une conduite d'entrée (48) et une conduite de sortie (50) du liquide à échantillonner entre lesquelles se trouve une capacité intermédiaire (52), la partie inférieure de ladite capacité intermédiaire (52) étant reliée à la conduite d'arrivée (48) par un conduit de vidange (54), la partie inférieure de l'embout (40) ayant la forme d'un manchon (56) plongeant dans ladite capacité intermédiaire (52) et ouvert à son extrémité inférieure (58), ledit support (46) présentant en outre un orifice (60) mettant en communication la conduite d'arrivée (48), la conduite de sortie (50) et le conduit de vidange (54) à un niveau inférieur à celui de la partie inférieure de la capacité intermédiaire (52).

4. Dispositif selon la revendication 1, caractérisé en ce que le dispositif de prélèvement (12) comprend :

– au moins une aiguille creuse montée sur un embout lui-même disposé sur un support (62),

– une conduite (66) sensiblement verticale ayant une extrémité supérieure reliée audit support (62) et une extrémité inférieure, et

– une capacité (68) apte à retenir une certaine quantité du liquide à échantillonner (70), la partie inférieure de ladite conduite (66) débouchant dans cette capacité (68), celle-ci étant en communication avec les conduites (34, 18) reliant le dispositif de prélèvement (12) à la capacité intermédiaire (32) et au séparateur (16), une partie de l'aiguille creuse se trouvant dans ladite conduite (66) et l'extrémité inférieure de cette aiguille se trouvant dans la masse de liquide (70) contenue dans ladite capacité (68) lorsque l'embout est monté sur le support (62).

**Patentansprüche**

1. Vorrichtung zur kontinuierlichen Zirkulation einer Flüssigkeit zwischen einem Vorratsbecken (10) und einer Entnahmeeinrichtung (12), welche in einer Höhe oberhalb des Spiegels der in dem Becken enthaltenen Flüssigkeit angeordnet ist, mit:

– einer Zufuhrleitung (14) für den Transport der Flüssigkeit zwischen dem Vorratsbecken (10) und der Entnahmeeinrichtung (12),

– einer Druckgasquelle (28),

– einer die Druckgasquelle (28) mit der Zufuhrleitung (14) verbindenden Leitung (30),

– einem ein oberes und ein unteres Teil aufweisenden Separator (16), wobei die Entnahmeeinrichtung (12) in einer über der des Separators (16) liegenden Höhe angeordnet ist,

– einer die Entnahmeeinrichtung (12) mit dem Separator (16) verbindenden Leitung (18),

– einer Einrichtung zum Erzeugen eines Unterdrucks,

– einer mit einem Tröpfchenabscheider (22) versehenen Leitung (20), welche das obere Teil des Separators (16) mit der Einrichtung zum Erzeugen des Unterdrucks verbindet,

– und einer das untere Teil des Separators (16) mit dem Becken (10) verbindenden Rückführleitung (24), dadurch gekennzeichnet, daß sie zusätzlich die folgenden Komponenten aufweist:

– einen als geschlossenen Behälter ausgebildeten Zwischenspeicher (32) mit einem oberen und einem unteren Teil, welche durch ein Mittelstück voneinander getrennt sind, wobei die Zufuhrleitung (14) am Mittelstück des Zwischenspeichers (32) in dessen Innenraum mündet und das untere Teil des Zwischenspeichers (32 in einer Höbe oberhalb der Entnahmeeinrichtung (12) angeordnet ist,

– eine das untere Teil des Zwischenspeichers (32) mit der Entnahmeeinrichtung (12) verbindende Leitung (34), und

– eine das obere Teil des Zwischenspeichers (32) mit der Einrichtung zum Erzeugen des Unterdrucks verbindende Leitung (36).

2. Vorrichtung nach Anspruch 1, dadurch gekennzeichnet, daß die das obere Teil des Zwischenspeichers (32) mit der Einrichtung zum Erzeugen des Unterdrucks verbindende Leitung (36) in der die Entnahmeeinrichtung (12) mit dem Separator (16) verbindenden Leitung (18) ausmündet.

3. Vorrichtung nach Anspruch 1, dadurch gekennzeichnet, daß die Entnahmeeinrichtung wenigstens eine Hohlnadel (38) aufweist, welche auf einer Zwinge (40) montiert ist, welche ihrerseits auf einer Fassung (46) angeordnet ist, mit einer Einlaßleitung (48) und einer Auslaßleitung (50) für die zur Entnahme einer Probe bestimmte Flüssigkeit, zwischen denen ein Zwischenspeicher (52) angeordnet ist, dessen unteres Teil über eine Entleerungsleitung (54) mit der Einlaßleitung (48) verbunden ist, wobei das untere Teil der Zwinge (40) die Form einer in den Zwischenspeicher (52) hineinragenden und am unteren Ende (58) offenen Buchse (56) aufweist und die Fassung außerdem einen Durchlaß (60) aufweist, welcher in einer unterhalb des unteren Teils des Zwischenspeichers (52) liegenden Höhe eine Strömungsverbindung zwischen der Einlaßleitung (48), der Auslaßleitung (50) und der Entleerungsleitung (54) bildet.

4. Vorrichtung nach Anspruch 1, dadurch gekennzeichnet, daß die Entnahmeeinrichtung (12) die folgenden Komponenten aufweist:
– wenigstens eine Hohlnadel, welche auf einer Zwinge montiert ist, welche ihrerseits auf einer Fassung (62) angeordnet ist,
– eine im wesentlichen senkrechte Leitung (66), welche ein mit der Fassung (62) verbundenes oberes Ende sowie ein unteres Ende aufweist, und
– einen Speicher (68) zum Speichern einer gewissen Menge der für die Entnahme einer Probe bestimmten Flüssigkeit (70), wobei das untere Ende der genannten Teilung (66) in dem Speicher (68) ausmündet und dieser mit den die Entnahmeeinrichtung (12) mit dem Zwischenspeicher (32) und dem Separator (16) verbindenden Leitungen (34, 18) strömungsverbunden ist, und wobei sich ein Teil der Hohlnadel in der genannten Leitung (66) befindet und das untere Ende der Nadel in die in dem Speicher (68) vorhandene Flüssigkeitsmenge (70) eintaucht, wenn die Zwinge auf die Fassung (62) aufgesetzt ist.

## Claims

1. System for continuously circulating a liquid between a storage vessel (10) and a sampling device (12) disposed at a level higher than the level of the free surface of the liquid contained in the vessel, comprising:
– a supply conduit (14) for conveying the liquid between the storage vessel (10) and the sampling device (12),
– a compressed gas source (28),
– a conduit (30) connecting the compressed gas source (28) to the supply conduit (14),
– a separator (16) having an upper portion and a lower portion, the sampling device (12) being at a higher level than the separator (16),
– a duct (18) connecting the sampling device (12) to the separator (16),
– an apparatus for setting up a negative pressure,
– a conduit (20) having a droplet remover (22) and connecting the upper portion of the separator (16) to the apparatus for setting up a negative pressure, and
– a return conduit (24) connecting the lower portion of the separator (16) to the vessel (10), characterized in that it also comprises:
– an intermediate capacity (32) formed by a closed receptacle, the intermediate capacity (32) having an upper portion and a lower portion separated by an intermediate portion, the supply conduit (14) discharging into the intermediate capacity (32) in its intermediate portion, the lower portion of the intermediate capacity (32) being disposed at a higher level than the sampling device (12),
– a conduit (34) connecting the lower portion of the intermediate capacity (32) to the sampling device (12), and
– a conduit (36) via which the upper portion of the intermediate capacity (32) communicates with the apparatus for setting up a negative pressure.

2. System according to claim 1, characterized in that, the last-mentioned conduit (36), via which the upper portion of the intermediate capacity (32) communicates with the apparatus for setting up a negative pressure, discharges into the duct (18) connecting the sampling device (12) to the separator (16).

3. System according to claim 1, characterized in that the sampling device comprises at least one hollow needle (38) mounted on a tip (40) disposed on a support (46) comprising an inlet conduit (48) and an outlet conduit (50) for the liquid to be supplied, between which an intermediate capacity (52) is disposed, the lower portion of the intermediate capacity (52) being connected to the inlet conduit (48) via an emptying duct (54), the lower portion of the tip (40) taking the form of a sleeve (56) immersed in such intermediate capacity (52) and open at its lower end (58), the support (46) also having an aperture (60) via which the inlet conduit (48), the outlet conduit (50) and the emptying duct (54) communicate at a level lower than that of the lower portion of the intermediate capacity (52).

4. System according to claim 1, characterized in that the sampling device (12) comprises:
– at least one hollow needle mounted on a tip disposed on a support (62),
– a substantially vertical conduit (66) having an upper end connected to the support (62) and the lower end, and
– a capacity (68) adapted to retain a certain quantity of the liquid to be sampled (70), the lower portion of such conduit (66) discharging into such capacity (68), the latter being in commnication with the conduits (34, 18) connecting the sampling device (12) to the intermediate capacity (32) and the separator (16), a portion of the hollow needle being disposed in said conduit (66) and the lower end of the needle being disposed in the body of liquid (70) contained in the last-mentioned capacity (68) when the tip is mounted on the support (62).

FIG. 1A

FIG. 1B

12

38

40

42

44

46

48

50

52

56

58

54

**FIG. 2**

12

38

40

48

54

60

50

**FIG. 3**

FIG. 4

FIG. 5 A

FIG. 5 B